# EUROPEAN PATENT SPECIFICATION

(11) **EP 4 398 262 B1**
(45) Date of publication and mention of the grant of the patent: **07.10.2026**
(21) Application number: 23215466.6
(22) Date of filing: 11.12.2023
(51) Int. Cl.: G16H 50/30, G16H 50/20, G16H 40/60, A61B 5/00, A61B 5/318, A61B 5/341, A61B 5/28, A61B 5/308, A61B 5/349, G16H 50/70

(54) **SYSTEM FOR DIAGNOSIS STRUCTURAL HEART DISEASE BASED ON ELECTROCARDOGRAM DATA**
SYSTEM ZUR DIAGNOSE STRUKTURELLER HERZERKRANKUNGEN AUF BASIS VON ELEKTROKARDIOGRAMMDATEN
SYSTÈME DE DIAGNOSTIC DE CARDIOPATHIE STRUCTURELLE BASÉS SUR DES DONNÉES D'ÉLECTROCARDIOGRAMME

(30) Priority: 03.01.2023 US 202318149540
(43) Date of publication of application: 10.07.2024
(73) Proprietor: GE Precision Healthcare LLC, Waukesha, WI 53188 (US)
(72) Inventor: ROWLANDSON, Gordon Ian, Wauwatosa, 53226 (US); YOUNG, Brian, Wauwatosa, 53226 (US)
(74) Representative: Kilburn & Strode LLP

(56) References cited:
- EP-A2- 2 438 848
- WO-A1-2022/117209
- US-A1- 2004 193 064
- US-A1- 2013 096 447
- US-A1- 2021 401 349
- US-A1- 2022 095 982
- US-A1- 2022 304 613
- US-A1- 2022 330 899
- SBROLLINI AGNESE ET AL: "Serial electrocardiography to detect newly emerging or aggravating cardiac pathology: a deep-learning approach", BIOMEDICAL ENGINEERING ONLINE, vol. 18, no. 1, 12 February 2019 (2019-02-12), GB, XP093123674, ISSN: 1475-925X, DOI: 10.1186/s12938-019-0630-9
- LEHMACHER JONAS ET AL: "Predictive Value of Serial ECGs in Patients with Suspected Myocardial Infarction", vol. 9, no. 7, 20 July 2020 (2020-07-20), CH, pages 2303, XP093124874, ISSN: 2077-0383, Retrieved from the Internet <URL:https://www.ncbi.nlm.nih.gov/pmc/articles/PMC7408822/pdf/jcm-09-02303.pdf> [retrieved on 20240126], DOI: 10.3390/jcm9072303
- DWIVEDI TRISHA ET AL: "Machine learning models of 6-lead ECGs for the interpretation of left ventricular hypertrophy (LVH)", JOURNAL OF ELECTROCARDIOLOGY., vol. 77, 13 December 2022 (2022-12-13), XX, pages 62 - 67, XP093124921, ISSN: 0022-0736, DOI: 10.1016/j.jelectrocard.2022.12.001
- ANONYMOUS HOLTER ET AL: "Holter Monitor | Johns Hopkins Medicine", 2 October 2022 (2022-10-02), pages 1 - 5, XP093277183, Retrieved from the Internet <URL:https://web.archive.org/web/20221002032650/https://www.hopkinsmedicine.org/health/treatment-tests-and-therapies/holter-monitor> [retrieved on 20250514]

## Description

### FIELD

Embodiments of the subject matter disclosed herein relate to medical devices, and in particular to systems for diagnosing progressive structural heart disease based on serial analysis of electrocardiogram (ECG) data.

### BACKGROUND

Electrocardiograph (ECG) monitors are widely used to obtain medical (e.g., biopotential) signals containing information indicative of electrical activity associated with the heart and pulmonary system of a patient. To obtain medical signals, ECG electrodes are applied to the skin of the patient in various locations. The electrodes, after being positioned on the patient, connect to an ECG monitor by a set of ECG lead wires. The ECG signals obtained with the electrodes may be processed into biopotential signals that may be evaluated by a clinician to diagnose or rule out various patient conditions.

Sbrollini Agnese et al: "Serial electrocardiography to detect newly emerging or aggravating cardiac pathology: a deep-learning approach" describes electrocardiogram (ECG) diagnosis by comparing the ECG under consideration with a previously made ECG in the same individual.

Lehmacher Jones et al: "Predictive Value of Serial ECGs in Patients with Suspected Myocardial Infarction" describes an aim to analyze the predictive value of ischemic ECG-signs in patients with suspected AMI. Patients presenting to the emergency department with suspected AMI were included. All patients with ST-elevation AMI were excluded from analyses.

### BRIEF DESCRIPTION

The scope of protection is limited to the appended claims. According to an aspect of the disclosure, a system for diagnosing a progressive structural heart disease may include a 6-lead electrocardiogram configured to generate 6-lead electrocardiogram (ECG) data, the 6-lead electrocardiograph including 3 electrodes and a monitor configured to measure electrical signals through the electrodes; a 12-lead electrocardiogram configured to generate 12-lead ECG data, the 12-lead electrocardiogram including 10 electrodes and a monitor configured to measure electrical signals through the electrodes; and an analysis device including a memory storing instructions and at least one processor configured to execute the instructions to: obtain, from the 12-lead ECG, 12-lead ECG data corresponding to a patient; analyze, by an AI algorithm, the 12-lead ECG data for signs of the progressive structural heart disease; based on the 12-lead ECG data indicating the disease: obtain, from the 6-lead ECG, multiple sets of 6-lead ECG data corresponding to the patient, the sets of 6-lead ECG data being obtained during spaced apart time intervals to show changes over time; performing serial analysis on the multiple sets of the 6-lead ECG data to validate the indicated progressive structural heart disease; controlling an operation of the detection device based on a result of the analysis.

According to another aspect of the disclosure, a system for diagnosing a progressive structural heart disease may include a memory storing instructions; and at least one processor configured to execute the instructions to: obtain first electrocardiogram (ECG) data corresponding to a patient, the first ECG data being obtained based on a first amount of leads; analyze, by an algorithm, the first ECG data for signs of the progressive structural heart disease; and based on the first ECG data indicating the disease: obtain multiple sets of second ECG data corresponding to the patient, the second ECG data being obtained based on a second amount of leads during spaced apart time intervals; perform serial analysis on the multiple sets of the second ECG data to validate the indicated progressive structural heart disease; and output a result of the serial analysis. The second amount of leads may be less than the first amount of leads.

According to yet another aspect of the disclosure, system for determining a degree of a progressive structural heart disease, the system may include a memory storing instructions; and at least one processor configured to execute the instructions to: obtain 12-lead electrocardiogram (ECG) data corresponding to a patient; analyze, by an algorithm, the first ECG data for signs of the progressive structural heart disease; and based on the first ECG data indicating the disease: obtain multiple sets of 6-lead ECG data corresponding to the patient, the second ECG data being obtained during spaced apart time intervals; obtain, from each set of the multiple sets of 6-lead ECG data, a first value corresponding to a first quantifiable feature; obtain a first trend value based on a first trend in the obtained first values; and obtain a degree score based on the first trend value, the degree score indicating a degree of the progressive structural heart disease.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description.

### BRIEF DESCRIPTION OF THE DRAWINGS

The present invention will be better understood from reading the following description of non-limiting implementations, with reference to the attached drawings, wherein below:
FIG. 1A shows a 12-lead ECG attached to a patient, according to an implementation.
FIG. 1B shows a 6-lead ECG being operated by a patient, according to an implementation.
FIG. 2 is a diagram of an electronic device that is a component of the system, according to an implementation.
FIG. 3 is a diagram of a network environment on which the system is operated, according to an implementation.
FIG. 4 is a diagram overviewing a system and method for diagnosing a patient based on serial analysis of ECG data, according to the invention.
FIG. 5 is a flowchart of process for diagnosing a patient based on serial analysis of ECG data, according to the invention.
FIG. 6 is a flowchart of a process of performing serial analysis on multiple sets of ECG data, according to an implementation.
FIG. 7 is a flowchart of a process of performing serial analysis on multiple sets of ECG data, according to an implementation.
FIG. 8 is a flowchart of a process of diagnosing a patient based on serial analysis of ECG data and patient symptom data, according to an implementation.

### DETAILED DESCRIPTION

The following detailed description of example implementations refers to the accompanying drawings. The same reference numbers in different drawings may identify the same or similar elements.

As used herein, the term "component" is intended to be broadly construed as hardware, firmware, or a combination of hardware and software.

It will be apparent that systems and/or methods, described herein, may be implemented in different forms of hardware, firmware, or a combination of hardware and software. The actual specialized control hardware or software code used to implement these systems and/or methods is not limiting of the implementations. Thus, the operation and behavior of the systems and/or methods were described herein without reference to specific software code-it being understood that software and hardware may be designed to implement the systems and/or methods based on the description herein.

No element, act, or instruction used herein should be construed as critical or essential unless explicitly described as such. Also, as used herein, the articles "a" and "an" are intended to include one or more items, and may be used interchangeably with "one or more" or "at least one." Furthermore, as used herein, the term "set" is intended to include one or more items (e.g., related items, unrelated items, a combination of related and unrelated items, etc.), and may be used interchangeably with "one or more." Where only one item is intended, the term "one" or similar language is used. Also, as used herein, the terms "has," "have," "having," "includes," "includes," "including," "comprises," "comprising," or the like are intended to be open-ended terms. Further, the phrase "based on" is intended to mean "based, at least in part, on" unless explicitly stated otherwise. References to "one implementation" of the present invention are not intended to be interpreted as excluding the existence of additional implementations that also incorporate the recited features. The terms "first," "second," and "third," etc. are used merely as labels, and are not intended to impose numerical requirements or a particular positional order on their objects.

The following description relates to various implementations of a system and method for diagnosing a patient based on serial analysis of electrocardiogram (ECG) data. The references to the methods of treatment by therapy or surgery or in vivo diagnosis methods are disclosed here only for the purpose of explaining the invention but do not fall within the scope of the claims. During a professional examination, the electrical activity of a patient's heart may be measured using a 12-lead ECG. The 12-lead ECG data may then be used to assist in diagnosing or predicting structural heart disease. For example, the 12-lead ECG data may be analyzed by an algorithm, such as an artificial intelligence (AI) based algorithm, that has been designed and/or trained to detect signs of progressive structural heart disease. However, the accuracy of these algorithms cannot be relied upon, thus additional testing is typically needed to diagnose a patient. In many cases, a transthoracic echocardiogram is ordered to validate the output of the algorithm. However, performing an echocardiogram each time the algorithm detects signs of structural heart disease in the ECG data has may drawbacks. For example, echocardiograms are resource intensive since they require an expensive machine that is operated by a trained professional and outputs information that must be analyzed by another trained professional. Additionally, obtaining an echocardiogram typically requires an additional appointment in which the patient must travel to a location having the resources necessary to perform the echocardiogram. Further, the results of an echocardiogram are not always indicative of a disease or a severity of the disease.

Obtaining additional 12-lead ECG data is also difficult for similar reasons as the echocardiogram. A 12-lead ECG is an expensive machine that is operated by a trained professional and outputs information that must be analyzed by another trained professional. Additionally, obtaining additional 12-lead ECGs requires additional appointments in which the patient must travel to a location having the resources necessary to perform the 12-lead ECG.

The system and method for diagnosing a patient based on serial analysis of ECG data may validate a disease detected in the 12-lead ECG data by an algorithm using a relatively inexpensive device that can be operated by a patient at home. Therefore, there is much less burden on the patient since he/she does not need to schedule and travel to additional appointment or pay large fees for professionals operating expensive equipment.

The system and method for diagnosing a patient based on serial analysis of ECG data may first analyze 12-lead ECG data for signs of a disease. For example, the 12-lead ECG data may be analyzed by an AI algorithm trained to detect structural heart disease in 12-lead ECG data. When the algorithm detects a sign of a disease, a validation process may be triggered to validate the detected disease. The validation procedure may obtain multiple sets of 6-lead ECG data from the patient at different times (e.g. once a week) and analyze the multiple sets of 6-lead ECG data for changes over time. The patient is able to obtain the 6-lead ECG data at home using a relatively inexpensive and easy to operate 6-lead ECGs, thus obviating the time and cost burdens associated with other methods.

The multiple sets of the 6-lead ECG data may be analyzed for tends over time. For example, quantifiable features can be logged for each set of ECG data and changes in the quantity can be tracked over the time period in which the ECGs were obtained (e.g. 3 months). Changes in ECG data over time can provide additional insights into the patient's condition than cannot be accurately obtained from looking at a single ECG recording. That is, the changes in the ECG data may provide more insight on a condition than analyzing just the features.

Changes in the ECG data that are indicative of a disease can be detected in lower lead ECG dad, such as 6-lead ECG data. As such, ECG data from lower lead amounts, such as 6-leads, can be used to accurately diagnose a patient, thus providing for the disclosed lower burden process of diagnosing a patient without multiple trips to professionals and large medical expenses.

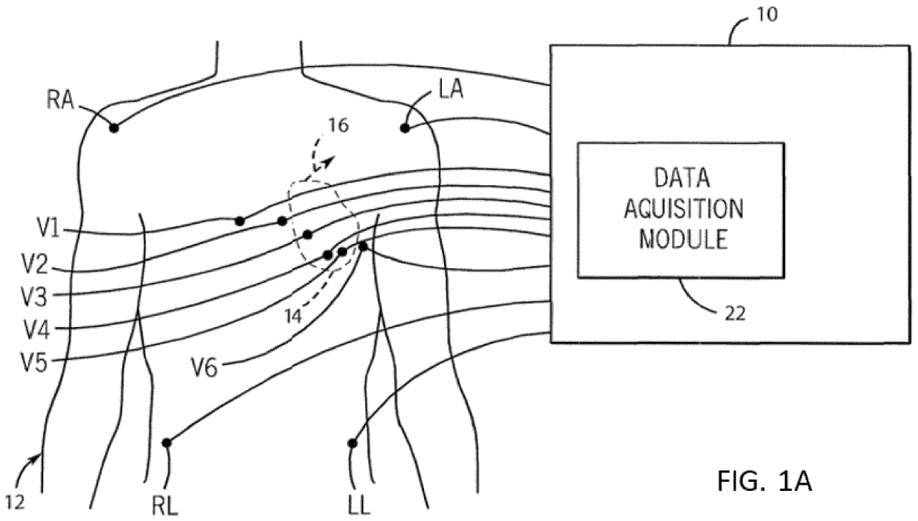

FIG. 1A shows a 12-lead ECG attached to a patient, according to an implementation. The 12-lead ECG 10 is connected to the patient 12 via a plurality of electrodes (labeled RA, LA, RL, LL, V1, V2, V3, V4, V5 and V6). The electrodes are typically placed at standard anatomical locations as in accordance with the standard acquisition of a 12-lead ECG. A 12-lead ECG utilizes four limb electrodes (RA, LA, RL, LL) and six precordial electrodes (V1, V2, V3, V4, V5, and V6). Differential voltages are obtained between two or more of these electrodes to obtain leads or projections through the heart along which the electrical characteristics of the heart are measured.

The heart 14 of the patient 12 produces an electrical impulse 16 at regular intervals. The electrical impulse causes the muscle tissue of the heart to contract as the electrical impulse 16 propagates through the heart. The resulting electrical potential generated at the patient's skin due to the propagation of this electrical impulse through the heart muscle tissue is measured by each of the electrodes and represented by each of the ECG leads.

The electrodes placed on the patient 12 are electrical transducers, and as such will pick up all electrical energy at the electrode location and provide the sensed electrical signals to the data acquisition module 22 of the monitoring system 10. Therefore, the data acquisition module 22 acquires not only the electrical heart impulses 16, but also electrical noise from any number of sources in and around the patient. The electrical noise may include electro-magnetic noise from man-made sources, electrical impulses from other medical devices used to provide care to the patient, and from other biopotentials generated by the patient such as muscle contractions (EMG) and/or brain waves (EEG).

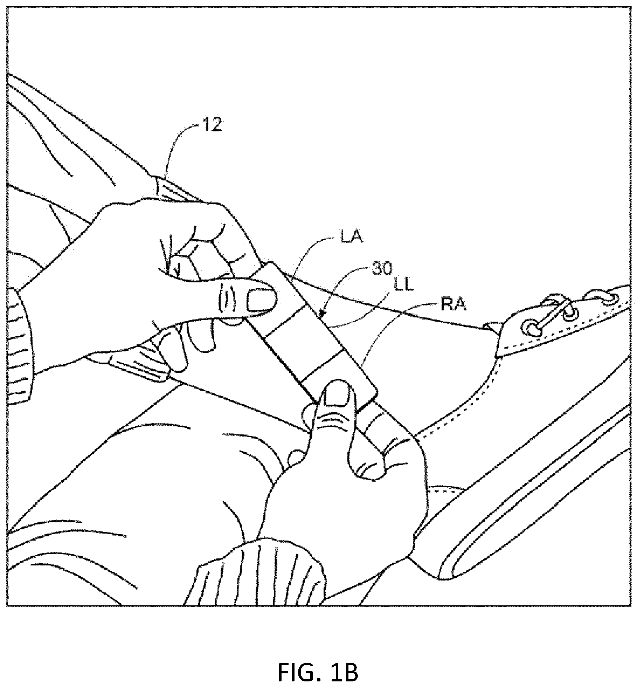

According to an implementation, the data acquisition module 22 may be included in an electrode monitor, also referred to herein as an ECG monitor, that receives the sensed combined signal and converts the signal from an analog signal into a digital signal. In implementations, the data acquisition module/ECG monitor is a high bandwidth module, wherein the combined signal sensed by the electrodes is digitized at a high sampling rate in comparison to standard ECG monitoring techniques. Standard ECG monitoring techniques typically implement a digitizing sampling rate between 500 and 1000 Hz. In a high bandwidth data acquisition module/ECG monitor, the combined signal is sampled at a rate of 50 kHz or higher. The combined signal may be sampled at a rate between 50-75 kHz. Alternatively, the combined signal may be sampled at a rate higher than standard ECG monitoring techniques but at a rate below the disclosed high bandwidth ECG monitoring technique. These alternative sampling rates may be any sampling rate above 1 kHz and below 50 kHz. In some implementations the high bandwidth of the combined signal provides increased signal data that facilitates the signal processing as described in further detail herein. Thus, the system shown in FIG. 1 includes a plurality of electrodes configured to measure electrical potential generated at a skin of a patient and an electrode monitor (e.g., an ECG monitor) configured to generate an electrocardiogram (ECG) signal (e.g., via the data acquisition module 22) from the electric potential measured by the plurality of electrodes.

A 12-lead ECG is typical operated by a medical professional in a medical environment. Since improper or inconsistent placement of electrodes can cause inaccurate readings, an operator of the device is trained to attach the electrodes based on anatomical landmarks on the patient.

FIG. 1B shows a 6-lead ECG being operated by a patient, according to an implementation. The 6-lead ECG 30 interfaces with the patient through the limb electrodes LA, RA, and LL. As shown in FIG. 1B, the patient may grip the LA electrode with a left hand and the RA electrode with a right hand. The LL electrode on the back of the device may be pressed into the left leg of the patient. The 6-lead ECG provides leads I, II, III, VR, aVL, and aVF. The 6-lead ECG obtains and process data in a similar manner as discussed above with respect to the 12 lead ECG.

As shown in FIG. 1B, the 6-lead ECG can be simply operated by a patient gripping the electrodes on both sides of the device and placing the back of the device on their left leg. The device may then take a measurement and send the data to a secondary device such as a smartphone where the data can be processed and displayed for the user or sent to another device over a network. As such, the 6-ECG is capable of being used operated by a patient at their home.

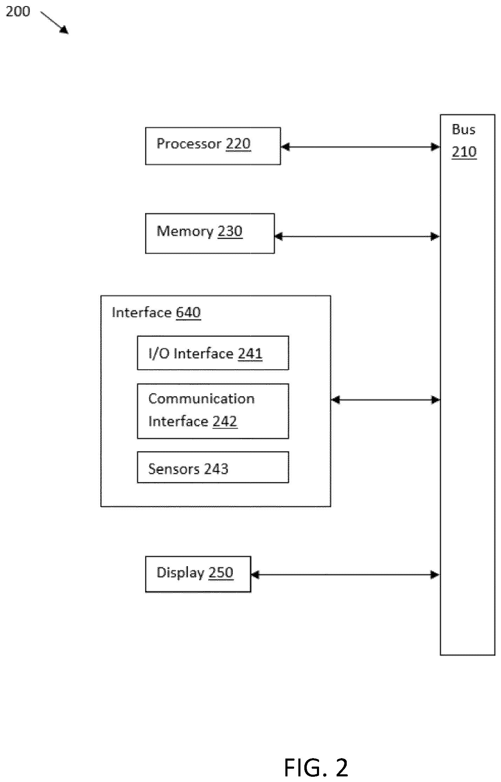

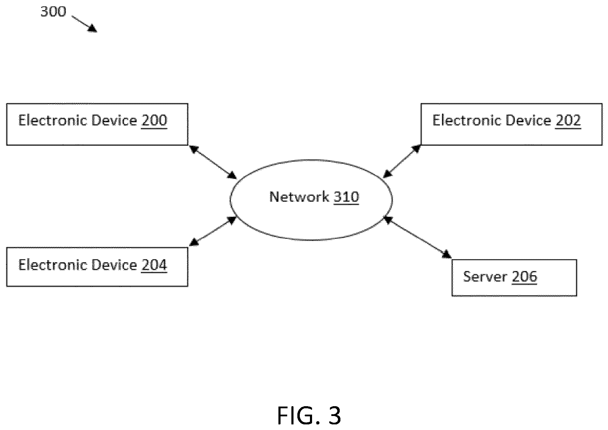

A method for diagnosing a patient based on serial analysis of ECG data may be deployed, partially deployed, operated, or accessed on electronic device 200 of Fig. 2 in a network environment 300 as shown in Fig. 3, according to an implementation. Figs 2 and 3 are for illustration only, and other implementations of the electronic device and environment could be used.

As shown in FIG. 2 electronic device 200 may include at least one of a bus 210, a processor 220 (or a plurality of processors), a memory 230, an interface 240, and a display 250.

Bus 210 may include a circuit for connecting the components 220, 230, 240, and 250 with one another. Bus 210 may function as a communication system for transferring data between the components, or between electronic devices.

Processor 220 may include one or more of a central processing unit (CPU), a graphics processor unit (GPU), an accelerated processing unit (APU), many integrated core (MIC), a field-programmable gate array (FPGA), or a digital signal processing (DSP). Processor 220 may control at least one of other components of electronic device 200, and/or perform an operation or data processing relating to communication. Processor 220 may execute one or more programs stored in memory 230.

Memory 230 may include a volatile and/or a non-volatile memory. Memory 230 may store information, such as one or more commands, data, programs (one or more instructions), or applications, etc., that is related to at least one other component of the electronic device 200 and for driving and controlling electronic device 200. For example, commands or data may formulate an operating system (OS). Information stored in memory 230 may be executed by processor 220.

The application may include one or more implementations as discussed above. These functions can be performed by a single application or by multiple applications that each carry out one or more of these functions.

Display 250 may include, for example, a liquid crystal display (LCD), a light emitting diode (LED) display, an organic light emitting diode (OLED) display, a quantum-dot light emitting diode (QLED) display, a microelectromechanical systems (MEMS) display, or an electronic paper display. Display 250 can also be a depth-aware display, such as a multi-focal display. Display 250 is able to present, for example, various contents (such as text, images, videos, icons, or symbols).

Interface 240 may include input/output (I/O) interface 241, communication interface 242, and/or one or more sensors 243. I/O interface 241 serves as an interface that can, for example, transfer commands or data between a user or other external devices and other component(s) of electronic device 200. The I/O interface 241 may provide a human-machine user interface which provides information to a user and receives information from the user.

Sensor(s) 243 may meter a physical quantity or detect an activation state of electronic device 200 and may convert metered or detected information into an electrical signal. For example, sensor(s) 243 may include one or more cameras or other imaging sensors for capturing images of scenes. The sensor(s) 243 may also include a microphone, a keyboard, a mouse, a touchscreen, one or more buttons for touch input, a gyroscope or gyro sensor, an air pressure sensor, a magnetic sensor or magnetometer, an acceleration sensor or accelerometer, a grip sensor, a proximity sensor, a color sensor (such as a red green blue (RGB) sensor), a bio-physical sensor, a temperature sensor, a humidity sensor, an illumination sensor, an ultraviolet (UV) sensor, an electromyography (EMG) sensor, an electroencephalogram (EEG) sensor, an electrocardiogram (EGG) sensor, an infrared (IR) sensor, an ultrasound sensor, an iris sensor, or a fingerprint sensor. The sensor(s) 243 can further include an inertial measurement unit. In addition, sensor(s) 243 can include a control circuit for controlling at least one of the sensors included here. Any of these sensor(s) 243 can be located within or coupled to electronic device 200. Sensor(s) 243 may be used to detect touch input, gesture input, hovering input using an electronic pen or a body portion of a user, etc.

According to a non-limiting implementation, the sensor(s) 243 may include a 12-lead ECG having 10 electrodes and a recorder to record electrical signal measured by the electrodes and/or a 6-lead ECG having 3 electrodes and a recorder to record electrical signals measured by the electrodes. The disclosure is not limited to these example ECG configurations and is intended to include ECGs having more or less electrodes and leads.

The communication interface 242 may communicate with one or more ECGs to obtain information from and/or control the ECG. The communication interface 242 may communicate with the ECG through a wired or wireless connection. The communication interface 242 may also communicate with a device storing ECG data to obtain the ECG data. The communication interface may also communication with an electronic medical record (EMR) database over a network.

Communication interface 242, for example, may be able to set up communication between electronic device 200 and an external electronic device (such as a first electronic device 202, a second electronic device 204, or a server 206 as shown in Fig. 3). As shown in Fig. 3, communication interface 242 may be connected with a network 310 through wireless or wired communication architecture to communicate with an external electronic device. Communication interface 242 may be a wired or wireless transceiver or any other component for transmitting and receiving signals.

Fig. 3 shows an example network configuration 300 according to an implementation. Electronic device 200 of Fig. 2 may be connected with a first external electronic device 202, a second external electronic device 204, or a server 206 through network 310. Electronic device 200 may be wearable device, an electronic device-mountable wearable device (such as an FIMD), etc. When electronic device 200 is mounted in the electronic device 202 (such as the FIMD), electronic device 200 may communicate with electronic device 202 through communication interface 242. Electronic device 200 may be directly connected with electronic device 202 to communicate with electronic device 202 without involving a separate network. Electronic device 200 may also be an augmented reality wearable device, such as eyeglasses, that include one or more cameras.

The first and second external electronic devices 202 and 204 and server 206 may each be a device of a same or a different type than electronic device 200. According to some implementations, server 206 may include a group of one or more servers. Also, according to some implementations, all or some of the operations executed on electronic device 200 may be executed on another or multiple other electronic devices (such as electronic devices 202 and 204 or server 206). Further, according to some implementations, when electronic device 200 should perform some function or service automatically or at a request, electronic device 200, instead of executing the function or service on its own or additionally, can request another device (such as electronic devices 202 and 204 or server 206) to perform at least some functions associated therewith. The other electronic device (such as electronic devices 202 and 204 or server 206) may be able to execute the requested functions or additional functions and transfer a result of the execution to electronic device 200. Electronic device 200 can provide a requested function or service by processing the received result as it is or additionally. To that end, a cloud computing, distributed computing, or client-server computing technique may be used, for example. While Figs 2 and 3 show that electronic device 200 including communication interface 242 to communicate with external electronic devices 202 and 204 or server 206 via the network 310, electronic device 200 may be independently operated without a separate communication function according to some implementations.

Server 206 may include the same or similar components 210, 220, 230, 240, and 250 as electronic device 200 (or a suitable subset thereof). Server 206 may support driving electronic device 200 by performing at least one of operations (or functions) implemented on electronic device 200. For example, server 206 can include a processing module or processor that may support processor 220 of electronic device 200.

The wireless communication may be able to use at least one of, for example, long term evolution (LTE), long term evolution-advanced (LTE-A), 5th generation wireless system (5G), millimeter-wave or 60 GHz wireless communication, Wireless USB, code division multiple access (CDMA), wideband code division multiple access (WCDMA), universal mobile telecommunication system (UMTS), wireless broadband (WiBro), or global system for mobile communication (GSM), as a cellular communication protocol. The wired connection may include, for example, at least one of a universal serial bus (USB), high definition multimedia interface (HDMI), recommended standard 232 (RS-232), or plain old telephone service (POTS). The network 710 includes at least one communication network, such as a computer network (like a local area network (LAN) or wide area network (WAN)), Internet, or a telephone network.

Although Fig. 3 shows one example of a network configuration 300 including an electronic device 200, two external electronic devices 202 and 204, and a server 206, various changes may be made to Fig. 3. For example, the network configuration 300 could include any number of each component in any suitable arrangement. In general, computing and communication systems come in a wide variety of configurations, and Fig. 3 does not limit the scope of this disclosure to any particular configuration. Also, while Fig. 3 shows one operational environment in which various features disclosed in this patent document can be used, these features could be used in any other suitable system.

The event detection method may be written as computer-executable programs or instructions that may be stored in a medium.

The medium may continuously store the computer-executable programs or instructions, or temporarily store the computer-executable programs or instructions for execution or downloading. Also, the medium may be any one of various recording media or storage media in which a single piece or plurality of pieces of hardware are combined, and the medium is not limited to a medium directly connected to electronic device 200, but may be distributed on a network. Examples of the medium include magnetic media, such as a hard disk, a floppy disk, and a magnetic tape, optical recording media, such as CD-ROM and DVD, magneto-optical media such as a floptical disk, and ROM, RAM, and a flash memory, which are configured to store program instructions. Other examples of the medium include recording media and storage media managed by application stores distributing applications or by websites, servers, and the like supplying or distributing other various types of software.

The method for diagnosing a patient based on serial analysis of ECG data may be provided in a form of downloadable software. A computer program product may include a product (for example, a downloadable application) in a form of a software program electronically distributed through a manufacturer or an electronic market. For electronic distribution, at least a part of the software program may be stored in a storage medium or may be temporarily generated. In this case, the storage medium may be a server or a storage medium of server 206.

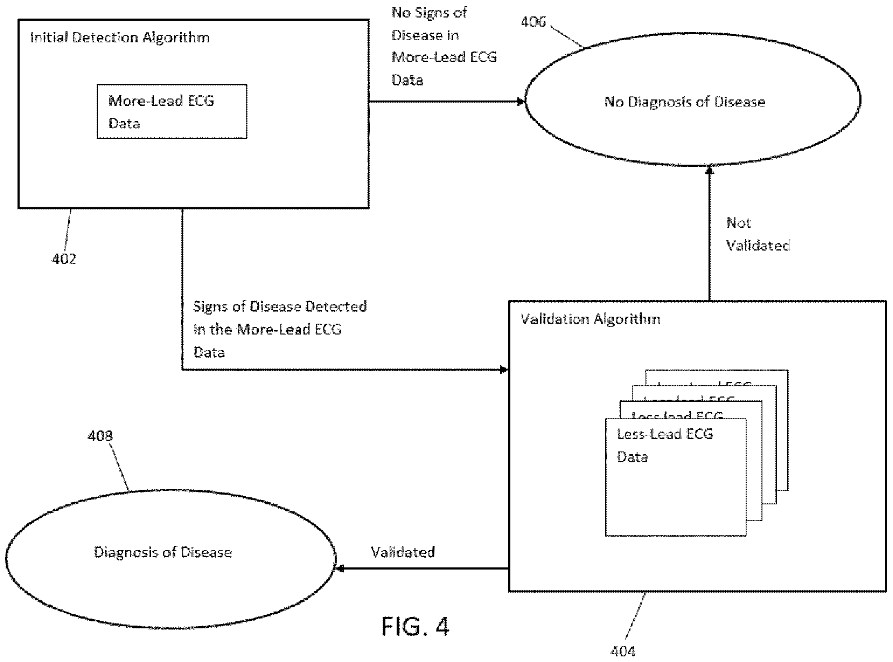

FIG. 4 is a diagram overviewing a system and method for diagnosing a patient based on serial analysis of ECG data, according to the invention.

As shown in element 402 of FIG. 4, more-lead ECG data is obtained from a patient. According to the invention, the more-lead ECG data is 12-lead ECG data. However, in other implementations not covered by the present invention, the more-lead ECG data is not limited to 12-lead ECG data and may include other lead configurations.

The more-lead ECG data may be analyzed by an initial detection algorithm that is trained to detect signs of heart conditions, such as structural heart disease (e.g. GE Healthcare's Marquette 12SL algorithm). The initial detection algorithm may analyze more-lead ECG data that was obtained over a time period, such as 30 seconds, and may output a detected condition, such as a specific type of structure heart disease. The initial detection algorithm for analyzing the more-lead ECG data is AI but is not limited to AI in other implementations not covered by the present invention.

In some cases, aspects of the ECG data that trigger the algorithm to detect signs of a disease are unknown, such a with an AI algorithm. Therefore, a trained care provider's review of the data often does not significantly increase an accuracy of the diagnosis. Accordingly, another means of validating the results of the AI algorithm may be used to further increase the accuracy of the diagnosis.

When the initial detection algorithm does not detect signs of a disease, the system may determine that there is no structural heart disease, as shown by element 406.

When the initial detection algorithm detects signs of a disease based on the more-lead ECG data, a care provider may direct the patient to provide additional ECG readings taken during spaced apart time intervals (e.g. once a week for 8 weeks) so changes in the ECG data over time can be observed. These changes can be used to validate the results of the initial detection algorithm.

Having multiple ECG recording over an extended period of time allows for the analysis of the recordings to focus on changes in the recordings or trends across the recordings. Trends across the multiple ECG recordings that are indicative of heart conditions are found using less-lead ECG data, that is 6-lead ECG data. As such, the 12-lead ECG data may not be necessary for detecting these trends. Accordingly, a less-lead ECG, that can be operated by a patient at home, can be used to obtain the multiple additional ECG readings.

As shown by element 404 of FIG. 4, multiple sets of less-lead ECG data are obtained. These sets of less-lead ECG data may be obtained at predetermined intervals from the patient using an at home device (e.g. once a week for 8 weeks). The patient uses an ECG having the 3 limb electrodes to provide the less-lead ECG data. The patient may then upload these multiple sets of less-lead ECG data to the cloud where they can be accessed for analysis.

A validation algorithm, which may be different than the initial detection that analyzed the more-lead ECG data, may be used to analyze the less-lead ECG data. The validation algorithm may detect a quantifiable metric in each ECG and may perform a serial comparison of the quantified metrics. For example, the validation algorithm obtains cardiac vectors from the multiple less-lead ECGs and detect trends in the cardiac vectors.

In a case in which the validation algorithm validates the disease, such as by detecting a trend that is indicative of the disease detected by the initial detection algorithm, the system may output a diagnosis of a disease, as shown by element 408. The diagnosis of the disease may indicate a severity of the disease. In a case in which the validation algorithm does not validate the disease, the system may output no diagnosis of a disease, as shown by element 406.

The system and method for diagnosing a patient based on serial analysis of ECG data provides for a patient to be accurately diagnosed without the need for implementing resource intensive and burdensome techniques (e.g. echocardiograms). Rather, the patient is able to be accurately diagnosed by data obtained at their home using a relatively inexpensive and simple to use device.

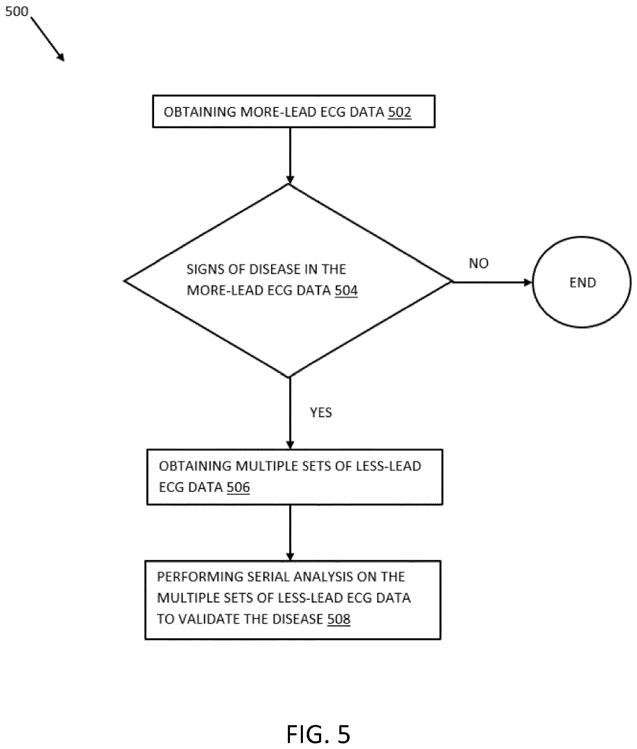

FIG. 5 is a flowchart of process 500 for diagnosing a patient based on serial analysis of ECG data, according to the present invention. The method of diagnosing a patient may include determining a degree of a chronic disease. According to the invention, the chronic disease is a progressive structural heart disease. Example diseases include pulmonary hypertension, pulmonary arterial hypertension, left ventricular hypertrophy, heart failure, low ejection fraction, and aortic stenosis, among others. The method 500 may be implemented on electronic devices 200 and/or server 206 in network environment 300.

At operation 502, more-lead ECG data is obtained. The more-lead ECG data is 12-lead ECG data obtained from a 12-lead ECG. According to some implementation, the more-lead ECG data may be data obtained using more leads than the less-lead ECG data. In the invention, more-lead ECG data is 12-lead ECG data and the less-lead ECG data is 6-lead ECG data. According to other examples not covered by the present invention, the more-lead ECG data may be 6-lead ECG data and the less-lead ECG data may be 1-lead ECG data. According to yet another example, the more-lead ECG data may be 15-lead ECG data and the less-lead ECG data may be 12-lead ECG data.

The more-lead ECG data may be raw data obtained by the electrodes of an ECG. According to some implementations, the raw data may be processed using techniques known in the art to process the raw ECG data, such as standardization, normalization, and or noise reduction techniques. The more-lead ECG data may include a continuous stream of time series ECG data obtained during a time period ranging from 1 second to 5 minutes. However, the disclosure is not limited to this range.

According to some implementations, the more-lead ECG data may be obtained from a database such as and EMR database. For example, a device performing method 500, such as device 200, may obtain the more-lead ECG data from an EMR database over a network.

At operation 504, the more-lead ECG data is analyzed for signs of a disease. According to an implementation, the more-lead ECG data may be analyzed by an algorithm known in the art that is designed to detect signs of a disease, such as structural heart disease. The algorithm uses artificial intelligence techniques such as machine learning and/or deep learning to detect signs of structural heart disease. The algorithm may output that a sign of a disease has been detected, as well as the specific disease (e.g. pulmonary hypertension) or a general condition (e.g. condition on right side of heart).

According to some implementations, the algorithm may input data from all the leads of the more-lead ECG data for the analysis. For example, when the more-lead ECG data is 12-lead ECG data, the algorithm may input and/or consider data from all 12 leads when performing the analysis.

Based on the disease not being detected by the algorithm, the process 500 may end, output data indicating that no disease was detected, or initiate another process. Based on signs of a disease being detected by the algorithm in the 12-lead ECG data, the process 500 proceeds to operation 506.

Operation 504 may be performed by a client device operated by a user performing the method 500, such as device 300. That is, the algorithm may be stored on the client device and the processor of the client device may perform the processing. According to other implementations, the algorithm may be stored on another device, such as an external server. In this implementation, the client device may send the more-lead ECG data to the external server, and the external server may run the data through the algorithm and return information corresponding to the output of the algorithm to the client device.

At operation 506, multiple sets of less-lead ECG data are obtained. The less-lead ECG data is 6-lead ECG data obtained from a 6-lead ECG. According to the invention, the less-lead ECG data is data obtained using less leads than the more-lead ECG data. In the invention, the less-lead ECG data is 6-lead ECG data and the more-lead ECG data is 12-lead ECG data. According to another example not covered by the present invention, the less-lead ECG data may be 1-lead ECG data and the more-lead ECG data may be 6-lead ECG data. According to yet another example, the less-lead ECG data may be 12-lead ECG data and the more-lead ECG data may be 15-lead ECG data.

The multiple sets of less-lead ECG data are obtained at spread apart time intervals. According to an implementation, the intervals during which the less-lead ECG is measured may be in the range of 2 second and 5 minutes, and the intervals may spaced apart by a time period of 1 day to 1 month. The above time ranges are provided for exemplary purposes and are not intended to be limiting.

According to an example, electric potentials of a patient's heart may be measured by a 12-lead ECG during a patient's a medical examination. In response to the analysis of the 12-lead ECG data indicating a disease, a care provider may order a patient to perform ECG measurements at set intervals, such as once a week for 2 months. The patient may then perform the measurements at home using an at home device, such as a 6-lead ECG designed to be operated by an untrained operator. According to another example, the patient may perform the ECG measurements at inconsistent intervals based on convenience. The cumulation of these ECG measurements may make up the multiple sets of less-lead ECG data. The less-lead ECG measurements may be uploaded to a network and/or cloud so they can be accessed by other devices.

Since the patient will likely not be performing the measurements at the exact intervals, each reading may be timestamped so the rate of change can be provided in a standardized format. For example, if a time period between first and second readings is 4 days, and a time period between second and third readings is 8 days, a change between the second and third readings may be halved to standardize the rate of change. That is, the non-standardized data can be converted to standardized data for analysis and comparison. The data may also be standardized using other method known in the art for providing the data is a comparable format.

According to some implementations, the multiple sets of less-lead ECG data may be obtained over a network from a database, such as an EMR database. For example, a patient may records the multiple less-lead ECGs at home and may upload the recordings to an EMR database where they can be accessed by a device performing process 500.

At operation 508, serial analysis is performed on the multiple sets of less-lead ECG data. The serial analysis may detect differences or trends in the less-lead ECG data. For example, a difference in a feature between each sequentially acquired ECG may be quantified, and a trend over time for the multiple sets of less-lead ECG data may be obtained. Many of these trends are indicative of a heart condition, such a structural heart disease.

Electrical forces generated by the heart can be thought of as a single vector. The summation of these instantaneous vectors during different phases of the cardiac complex can be used for generating an overall vector for that portion. According to an implementation, one or more cardiac vectors may be calculated for each set of the less-lead ECG data. The one or more cardiac vectors may include a mean QRS vector based on the QRS loop and a mean T-wave vector based on the T loop. The cardiac vectors may be obtained using known methods in the art.

By having multiple sets of ECG data that are taken over an extended time period, the progressive changes in the vectors may be tracked over time. These changes may then be used to diagnose a heart condition and/or determine a severity of a heart condition.

For example, in a healthy heart under normal conditions, the mean QRS vector and the mean T-wave vector point in the same direction. In the case of pulmonary hypertension, undue stress is put on the right side of the heart which causes the mean QRS vector to point rightward and anteriorly. Accordingly, pulmonary hypertension can be diagnosed based on the multiple sets of less-lead ECG data showing the mean QRS vector moving rightward on the frontal plane. Additionally, the rate or amount of movement can be analyzed to determine a severity of structural heart damage associated with the pulmonary hypertension.

In the case of a chronic disease such as ventricular hypertrophy due to hypertension, undue stress is put on the left side of the heart. When this occurs, the mean QRS vector points leftward and posteriorly. Accordingly, chronic hypertension can be diagnosed based on the multiple sets of less-lead ECG data showing the mean QRS vector moving leftward on the frontal plane. Additionally, the rate or amount of movement can be analyzed to determine a severity of structural heart damage associated with the chronic hypertension.

In both of the above conditions, pulmonary and chronic hypertension, the mean T-wave vector moves away from the mean QRS vector. A larger angle between the mean QRS vector and the mean T-wave vector (QRS-T angle) indicates a more severe disease. As such, the size of the QRS angle and the rate at which it is expanding can be tracked using the less-lead ECG data and can then be used to diagnose a patient.

Features other than cardiac vector, which are detectable in 6-lead ECG data may also be used diagnose heart conditions. Leads I, II, III, aVR, aVL, and aVF (6-lead ECG) are useful for detecting the evolution of the very chronic diseases that the AI-based algorithms for the 12-lead ECG data were originally intended for, namely pulmonary hypertension, left ventricular hypertrophy (LVH) due to chronic hypertension, or LVH due to aortic stenosis. Each of these chronic diseases puts undue stress on either the right or left side of the heart. Although it is not exactly clear what the AI algorithms are sensing in the 12-lead ECG data, there are conventional measures of the ECG known to be related to detecting chronic stress of either the right or left side of the heart which can be detected using a 6-lead ECG data.

For example, pulmonary hypertension stresses the right side of the heart. A number of indicators (like right bundle branch block or a prominent R-wave in V1) may be obtained from a chest lead (e.g. more-lead ECG data). When looking at only limb leads (e.g. less-lead ECG data), the conventional measures for pulmonary hypertension may include the following: right axis deviation; a tall P-wave in lead II; S-waves in lead I, II and III; a Q-wave with T-wave inversion in lead III and prolonged QT. Although these specific measurements are not predictive enough of pulmonary hypertension versus the general population, they are likely very predictive when used against the patient's own baseline recording (a trend over time). As such, when pulmonary hypertension is detected in the initially analyzed more-lead ECG data, it can be validated using the multiple sets of less-lead ECG data which show a trend or change from a baseline.

In contrast to pulmonary hypertension, chronic hypertension puts undue stress on the left side of the heart. There are number of indicators of this phenomena present in the chest leads (e.g. more-lead ECG data) as specified by conventional criteria that have been evaluated versus echocardiogram or cardiac magnetic resonance imaging. When considering the limb leads (e.g. less-lead ECG data), the following conventional measures are pertinent: leftward QRS axis deviation, P wave axis < 30°, positive P-wave terminal force in aVL, mitral P / P-wave duration in lead II, large R-wave in aVL, QRS duration, and ST segment depression in lateral leads. The limb leads can also risk stratify patients due to the consequences of stresses on the left side, such as measures of the P-wave and risk of atrial fibrillation /stroke or measures of QRS duration /ST depression and the onset of heart failure. As such, when chronic hypertension is detected in the initially analyzed more-lead ECG data, it can be validated using the multiple sets of less-lead ECG data.

Additionally, ST segment and T-wave inversion due to LVH / AS can also be tracked in the 6 lead ECG by using aVL. Furthermore, by using the patient's own ECG as their baseline, measurements obtained from the frontal plane can be useful for addressing this need.

Operation 508 may be performed by a client device operated by a user performing the method 500, such as device 300. That is, the processor of the client device may perform the serial analysis.

According to some implementations, process 500 may be performed by a background server, such as server 206. That is, the background server may obtain the ECG data from databases, perform the analysis on the data, and then store the results in memory or output the results to another device.

As discussed above, serial analysis of the less-lead ECG data can indicate a specific condition and a severity of the condition. Accordingly, the process 500 can output a specific condition and/or a severity of the condition. According to some implementations, the result of the serial analysis may be a portion of the heart suffering from a condition. For example, the serial analysis may determine the right side or the left side of the heart is functioning improperly. A care provider may then use this information in diagnosing the patient.

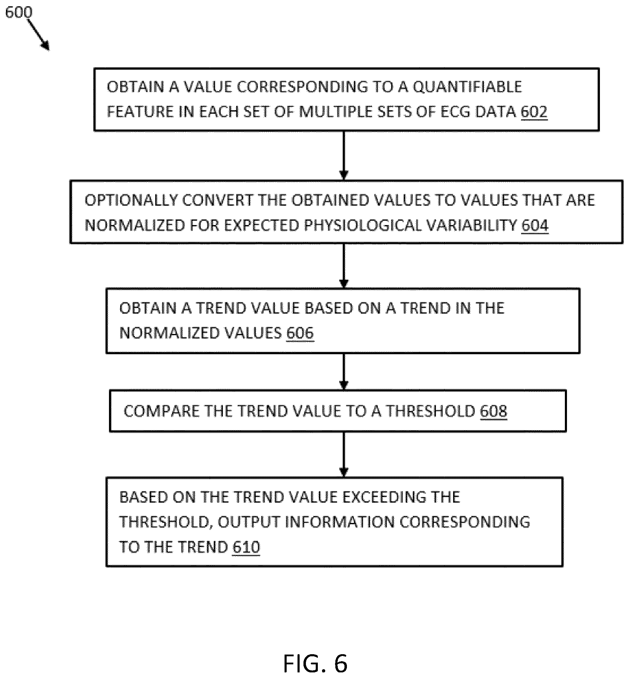

FIG. 6 is a flowchart of a process 600 of performing serial analysis on multiple sets of ECG data, according to an implementation. The method may be implemented on electronic device 200 and/or server 206 in network environment 300.

The multiple sets of ECG data, such as 6-lead ECG data, may be obtained during spaced apart time intervals. According to a non-limiting example, a patient may obtain the multiple sets of ECG data by recording an ECG measurement for a time intervals of 30 seconds once a week (i.e. time intervals spaced apart by a week). Each 30 second recording may be one set of ECG data, and one month worth of recordings (i.e. 4 recordings) may be the multiple sets of ECG data. According to an implementation, each recording may be obtained during a different episode of care.

At operation 602, a value corresponding to a quantifiable feature may be obtained from each set of ECG data. Each set of ECG data may be analyzed by an algorithm configured to detect a quantifiable feature in the ECG data. The feature may include the following non-limiting examples: a size of a cardiac vector (e.g. mean P-wave vector, mean QRS vector, mean T-wave vector), a direction/axis of a cardiac vector, size and/or shape of a P-wave, size and/or shape of a Q-wave, size and/or shape of an R-wave, size and/or shape of an S-wave, size and/or shape of a T-wave, timespan between any of the ECG waves, timespan between any features or points in the ECG data, or the difference or ratio between two related measurements (for example the difference between the mean QRS vector and the mean T-wave vector or the ratio between R-wave amplitude and S-wave amplitude in the QRS complex).

Once the quantifiable feature is detected, the feature may be quantified to obtain a value. For example, for a cardiac vector, the value may be coordinates indicating a direction and size of the cardiac vector. According to another example, for a QT interval, the value may a time in seconds between a Q-wave and a T-wave of a heartbeat.

The features may be detected and/or quantified by methods known in the art, such as one or more algorithms designed to detect features in ECG data. According to some implementations, the algorithms may use AI. According to other implementations, the algorithms may not use AI and may rather be based on preset rules.

According to some implementations, the value for each set of ECG data may be obtained based on multiple features detected in the ECG data. For example, a probability of pulmonary hypertension may be estimated based on the following Equation 1: [Equation 1] $\begin{matrix}Max of \left\{0 or \left(\left(QRS axis-\left(100-Age\right)/2\right)\right)\right\} + \\ \left(\left(\left(P-wave amplitude μV lead II\right)/250\right)\times 10\right) + \left(\left(QRS axis-T axis\right)/5\right)\end{matrix}$

In Equation 1, QRS axis° is the mean QRS cardiac vector in degrees, T axis is the mean T-wave cardiac vector in degrees, sand P-wave amplitude µV lead II is the amplitude of the P-wave in micro-volts. Since this equation determines a probability, the answer is bound between 0 and 100.

At operation 604, the obtained values may be optionally normalized for expected physiological variability. The ECG data for a patient may vary from day to day or even from minute to minute due to physiological variances. To obtain consistent data, the values may be normalized to remove at least a potion of the physiological variability from the values.

For example, the QT time interval may be affected by heart rate. A patient having a properly functioning heart may have a shorter QT time interval when their heart rate is elevated. This deviation in QT interval, which is due to physiological variance and not a heart condition, may result in an inaccurate diagnosis. As such, the value obtained during the elevated heart rate may be adjusted based on the heart rate so it can be more accurately compared to a QT interval taken during a normal heart rate. This normalization may be performed autonomously based on the elevated heart rate being detected. According to other implementations, the normalization may be performed in response to a received normalization value indicating an amount to change the raw value due to the physiological variance.

Amplitudes of an ECG signal may be impacted by may factors such as fluid in the lungs, a previous meal, temperature, ect. However, a direction of cardiac forces may be less affected by physiological variances and therefore may be a better indicator for determining a condition over time. Direction of forces are indicative by types & numbers of waves detected as well as the frontal plane axis measurements for P, QRS and T as well as spatial relationships (angles) between these axis.

At operation 606, a trend value may be obtained based on a trend in the values obtained from the ECG data and/or the normalized values. The trend may be obtained based on a serial comparison of the obtained values over time. Obtaining the trend value may include obtaining a list containing a difference between the variables of sequential ECG sets and obtaining a trend in the list. The resultant trend value may correspond to a change in the feature associated with the obtained values over time. According to an implementation, the trend in the values may then be quantified using a best fit trendline. According to other implementations, the trend may be obtained using other methods known in the art.

For example, when the obtained values correspond to an angle of the mean QRS vector, a trend may be a direction and a rate in which the mean QRS vector angle is moving over the multiple sets of ECG data. For the following data - increase of 2 degrees in week 1, increase of 1 degree in week 2, and increase of 3 degrees in week 3 - the trend value could be positive 2 degree per week.

According to another example, when the obtained values correspond to QT time interval, a trend may be a rate of an increase or decrease in the time interval. According to another example, when the trend values correspond to a probability of pulmonary hypertension, a trend may be a rate of increase or decrease in the probability.

According to an implementation, a score indicating a degree of the disease may be obtained from the trend value. For example, the score may be a number between 0 and 100. Since different features may trend at varying degrees, and a clinical significance of a severity of a trend is feature specific, the trend values may be standardized to a value between 0 and 100. For example, if a mean QRS vector angle may trend in the range of 0 degrees/week to 20 degrees per week, the trend value may be standardized to the 0 though 100 range by multiplying the trend value by 5. If a QT interval may trend in the range of 0 seconds per week to 0.1 second per week, the trend value may be standardized to the 0 though 100 range by multiplying the trend value by 1000. As such, each trend value may have a corresponding standardization factor that may be applied to standardize the trend value to the 0 though 100 range for simple analysis by a care provider. Further, a score indicating a degree of a disease may be obtained based on multiple trends values. For example, each trend value may be standardized to the 0 though 100 range and then a weighted average of the standardized values may be obtained. The weights may be assigned based on the significance of the features corresponding to the trend values.

At operation 608, the trend value may be compared to a threshold. Not all trends may be severe enough to warrant clinical intervention. To avoid providing the care team with unnecessary information, the trend may be compared to a threshold to determine if it is clinically significant. The threshold may be set by a care provider or may be determined based on patient data such as age, gender, race, preexisting conditions, ect.

For example, when tracking mean QRS vector angle, a threshold for a 60 year old man may be set to .8 degrees per week of change. This threshold may be set by a care provider based on information obtained by the care provider during an examination. Alternatively, this threshold may be set based on a predetermined lookup table in which the patient's information is input, such as gender, age, and preexisting conditions. The above example mean QRS vector data which includes an upward trend of 1 degree per week exceeds this threshold, thus indicating that the trend is clinically significant. Based on this determination, information corresponding to the trend can be presented to the care team or uploaded to a server or database. For example, a care provider and/or patient may receive a notification that the trend in the patient's data is clinically significant and requires further attention. This information may also be stored in a EMR databased for reference by others.

According to some implementations, multiple values may be obtained for each set of ECG data and multiple sets of trends values may be obtained and compared to thresholds.

According to some implementations, the trend value may be input into a diagnosis algorithm that is designed and/or trained to detect a disease based on trends in ECG data. For example, a feature and a trend value may be input into the algorithm and the algorithm may output a diagnosis based on the input information.

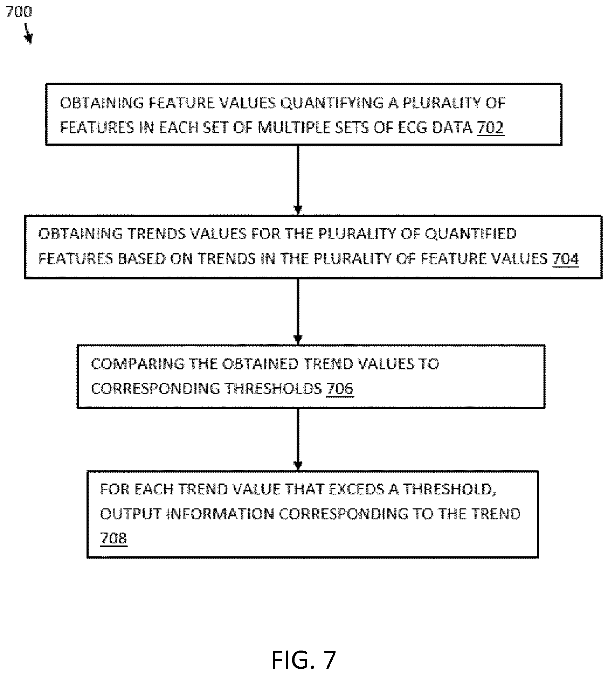

FIG. 7 is a flowchart of a process of performing serial analysis on multiple sets of ECG data, according to an implementation. The method may be implemented on electronic device 200 and/or server 206 in network environment 300.

At operation 702, a plurality of features may be quantified in each set of multiple sets of ECG data. The features may include one or more of the following non-limiting examples: a size of a cardiac vector (e.g. mean P-wave vector, mean QRS vector, mean T-wave vector), a direction/axis of a cardiac vector, size and/or shape of a P-wave, size and/or shape of a Q-wave, size and/or shape of an R-wave, size and/or shape of an S-wave, size and/or shape of a T-wave, timespan between any of the ECG waves, and timespan between any features or points in the ECG data.

For example, a feature value may be obtained for each of a mean P-wave vector angle, mean QRS vector angle, mean T-wave vector angle, QRS-T angle, and a QT interval. For the cardiac vectors, the feature value may be an angle of the vector on the frontal plane. For the QT interval, the feature value may be an amount of time in seconds.

At operation 704, trends values that indicate a trend over time may be obtained for the plurality of features based on trends in the plurality of feature values. An independent trend may be obtained for each feature by independently analyzing the feature values corresponding to the feature.

For example, the mean P-wave vector angle trend value may be positive 1.2 degrees/week (i.e. increasing 1.2 degrees per week on average), the mean QRS vector angle trend value may be 1.8 degrees/week for, the mean T-wave vector angle trend value may be 1.5 degrees/week, the QRS-T angle may be 1 degree/week, and the QT interval may be -0.01 seconds/week.

At operation 706, the obtained trend values may be compared to thresholds. The thresholds may be set based on clinical significance. According to a non-limiting implementation, a threshold may be set according to a predefined table which inputs patient information. According to another non-limiting implementation, the threshold may be set by a care provider. According to another implementation, the care provider may be presented with the thresholds provided by the table based on the patient's information, and the care provider may adjust the thresholds based on the care provider's knowledge of the patient.

For example, when considering the above example trends and the following thresholds -- 1 degrees/week for mean P-wave vector angle, 2 degrees/week for mean QRS vector angle, 2 degrees/week for mean t-wave vector angle, 1 degree/week for QRS-T angle, and 0.01 seconds/week for QT interval - the process 700 would output the trend values for mean P-wave vector and QRS-T angle since they exceed the threshold indicating they are of clinical significance. The trends values that do not exceed the threshold may not be output since they are deemed clinically insignificant or not valuable to a care provider.

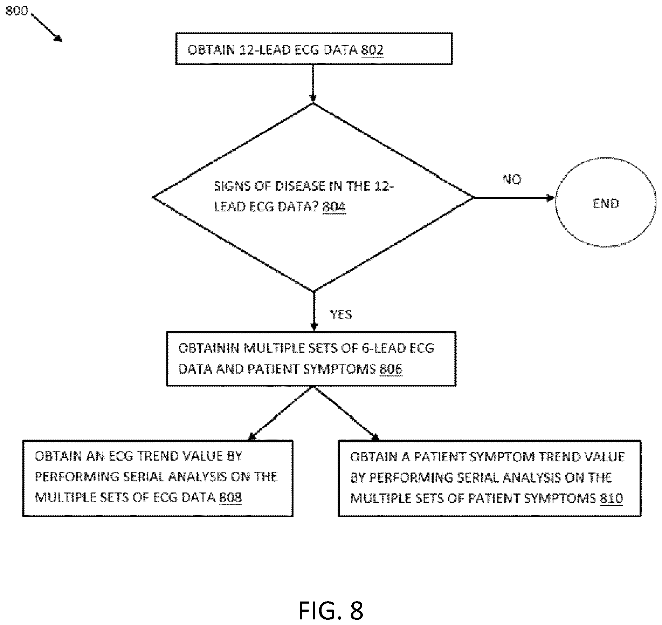

FIG. 8 is a flowchart of a process 800 of diagnosing a patient based on serial analysis of ECG data and patient symptom data, according to an implementation. The method may be implemented on electronic device 200 and or server 206 in network environment 300.

At operation 802, ECG data may be obtained from a patient. According to a non-limiting implementation, the ECG data may be measured from the patient using a 12-lead ECG. The ECG data may then be stored in a database, such as an EMR, where it can be obtained when process 800 is performed.

At operation 804, the 12 lead ECG data may be analyzed for signs of a disease. According to an implementation, the 12 lead ECG data may be analyzed by an algorithm known in the art that is designed to detect signs of a disease, such as structural heart disease.

Based on the algorithm detecting a sign of a disease in the 12 lead ECG data, the process 800 may progress to operation 806. Based on the algorithm not detecting a sign of a disease in the 12 lease ECG data, the process 800 may end, output data indicating that no disease was detected, or initiate another process.

At operation 806, multiple sets of ECG data and multiple sets of patient symptoms may be obtained. According to a non-limiting implementation, the multiple sets of ECG data may be obtained from an at home device, such as a 6 lead ECG. Each time the patient takes and ECG, the patient may also input symptoms being experienced at the time into a device that is also communicating with the ECG. The ECG data and symptoms obtained at the same time may be timestamped with the same time or related in another manner to indicate that they were obtained at the same time. The data may then be uploaded to a database. According to an implementation, a background server may perform operation 806 by accessing a database sorting the ECG and patient symptom data.

According to an implementation, patient's symptoms may be input by a patient using natural language. For example, the patient may input the following statement "I lack energy and feel a little nauseous." According to another implementation, patient symptoms may be selected from menus which also provides for a selection of a severity of each symptom. For example, the patient may select "lacks energy" for the symptom and may select "5" for severity.

For example, a patient may take an ECG once a week for 4 weeks. Each time the patient takes an ECG, the patient may be prompted by a device recording the ECG to input symptoms. At the end of the 4 weeks the patient will have 4 ECG recordings and 4 sets of associated symptoms.

At operation 808, ECG trend value(s) for the ECG data may be obtained by performing serial analysis on the multiple sets of ECG data. This operation may be performed similar to operation 508 of process 500. According to some implementations, multiple ECG trends values may be obtained for different quantifiable features in the ECG data.

At operation 810, patient symptom trend(s) value may be obtained in the multiple sets of patient symptom data. In order to obtain a trend value for a symptom, the severity of the symptom must be quantified. For the implementation in which the patient selects a symptom and severity, the severity of the symptom is quantified by the patient. For the implementations in which the patient inputs natural language, natural language processing techniques may be used to obtain symptoms and quantify a severity of each symptom. When considering the natural language input "I lack energy and feel a little nauseous," the natural language processing may determine "lack of energy" having a severity of "5", since the patient did not further specify their lack of energy, and "nauseous" having a severity of "2" since the patient used "little" before "nauseous."

Once the severities of the symptoms are quantified, a trend over time can be obtained for each symptom through serial analysis of the quantified symptom data.

The patient symptom trend(s) and ECG trend(s) may then be output by the process. According to an implementation, the trend data may be output to a care provider based on the trend value exceeding a threshold. According to an implementation, the trend data may be uploaded to a server or database for remote access. For example, the trends data may be accessed by an algorithm designed to diagnose a patient or determine a severity of a disease based on the trend data.

In the above exemplary implementations, ECG data is described as the data sensed from the patient and the data is being used to detect heart disease. The present disclosure is not limited to ECG data and heart disease detection. Rather, the disclosed processes of serial analysis may be performed using other physiological parameters and for detecting condition other than heart disease. For example, physiological parameters such as blood pressure, heart rate, blood glucose, blood oxygen, as well as other, can be tracked over time to obtain trends that may be indicative of diseases.

All of the values in the above examples are provider for explanatory purpose and are not intended to be limiting or to emulate real work data.

The scope of protection is limited to the appended claims.

## Claims

1. A system for diagnosing a progressive structural heart disease, comprising:
a 12-lead ECG configured to generate 12-lead ECG data, the-12 lead ECG comprising:
10 electrodes; and
a monitor configured to measure electrical signals through the electrodes;
a 6-lead electrocardiogram, ECG, configured to generate 6-lead electrocardiogram data, the 6-lead ECG comprising:
3 electrodes; and
a monitor configured to measure electrical signals through the electrodes;
and
an analysis device comprising:
a memory (230) storing instructions; and
at least one processor (220) configured to execute the instructions to:
obtain, from the 12-lead ECG attached to a patient, 12-lead ECG data corresponding to a patient;
analyze, by an AI algorithm, the 12-lead ECG data for signs of the progressive structural heart disease;
based on the 12-lead ECG data indicating the progressive structural heart disease:
obtain, from the 6-lead ECG attached to the same patient, multiple sets of 6-lead ECG data corresponding to the patient, the multiple sets of 6-lead ECG data being obtained during spaced apart time intervals to show changes over time;
perform serial analysis on the multiple sets of the 6-lead ECG data to validate the indicated progressive structural heart disease;
determining one or more trends across the multiple sets the 6-lead ECG data by:
obtaining, a first cardiac vector for each set of the 6-lead ECG data;
recording an angle of each of the first cardiac vectors,
obtaining a first diagnosis based on the angles of the first cardiac vectors trending in a first direction; and
obtaining a second diagnosis based on the angles of the first cardiac vectors trending in a second direction;
and
control an operation of the analysis device based on a result of the analysis.

2. The system of claim 1, wherein the at least one processor is configured to execute the instructions to, based on the 12-lead ECG data indicating the disease:
obtain, for each set of the 6-lead ECG data, a second cardiac vector;
obtain, for each set of the 6-lead ECG data, an angular relationship between the first vector and the second vector; and
detect a trend in the angular relationships.

3. The system of claim 1 or 2, wherein the at least one processor is configured to execute the instructions to, based on the 12-lead ECG data indicating the disease:
obtain, from each set of the multiple sets of 6-lead ECG data, a first value corresponding to a first quantifiable feature;
obtain a first trend value based on a trend in the first values; and generate a value indicating a degree of a disease based on the first trend value.

4. The system of claim 3, wherein the at least one processor is configured to execute the instructions to, based on the 12-lead ECG data indicating the disease:
compare the first trend value to a predefined threshold; and
based on the first trend value exceeding the predefined threshold, output the first trend value.

5. The system of claim 4, wherein the at least one processor is configured to execute the instructions to, based on the 12-lead ECG data indicating the disease:
obtain, from each set of the multiple sets of 6-lead ECG data, a second value indicating a second quantifiable metric;
detect a second trend in the obtained second values; and
generate a value indicating the degree of the disease based on the detected first trend and the detected second trend.

6. The system of claim 5, wherein the first trend value is based on a first feature in the 6-lead ECG data and the second trend values is based on a second feature in the 6-lead ECG data.

7. The system of any preceding claim, wherein the at least one processor is configured to execute the instructions to, based on the 12-lead ECG data indicating the disease, obtain a diagnosis by inputting the results of the serial analysis into an algorithm configured to diagnose progressive structural heart disease based on trends in 6-lead ECG data.

8. The system of any preceding claim, wherein the at least one processor is configured to execute the instructions to, based on the 12-lead ECG data indicating the disease:
obtain patient symptom data corresponding to each set of the multiple sets of the 6-lead ECG data.

9. The system of claim 8, wherein the at least one processor is configured to execute the instructions to, based on the 12-lead ECG data indicating the disease:
obtain a symptom trend value based on a serial analysis of one or more patient symptoms corresponding to the multiple sets of 6-lead ECG data to detect a trend in the patient symptoms over time.

10. The system of claim 9, wherein the at least one processor is configured to execute the instructions to, based on the 12-lead ECG data indicating the disease, obtain a diagnosis by inputting the results of the serial analysis of the 6-lead ECG data and a result of the serial analysis of the patient symptom data into an algorithm configured to diagnose progressive structural heart disease based on a trend in 6-lead ECG data and a trend in the patient symptoms.

## Patentansprüche

1. System zur Diagnose einer progressiven strukturellen Herzerkrankung, umfassend:
ein 12-Kanal-EKG, das zum Generieren von 12-Kanal-EKG-Daten ausgelegt ist, wobei das 12-Kanal-EKG Folgendes umfasst:
10 Elektroden; und
einen Monitor, der dazu ausgelegt ist, elektrische Signale durch die Elektroden zu messen;
ein 6-Kanal-Elektrokardiogramm, EKG, das zum Generieren von 6-Kanal-Elektrokardiogrammdaten konfiguriert ist, wobei das 6-Kanal-EKG Folgendes umfasst:
3 Elektroden; und
einen Monitor, der dazu ausgelegt ist, elektrische Signale durch die Elektroden zu messen;
und
eine Analysevorrichtung, umfassend:
einen Speicher (230), der Anweisungen speichert; und
mindestens einen Prozessor (220), der dazu ausgelegt ist, die Anweisungen auszuführen, zum:
Erhalten von 12-Kanal-EKG-Daten, die einem Patienten entsprechen, aus dem an einen Patienten angeschlossenen 12-Kanal-EKG;
Analysieren der 12-Kanal-EKG-Daten anhand eines KI-Algorithmus auf Anzeichen einer progressiven strukturellen Herzerkrankung;
basierend auf den 12-Kanal-EKG-Daten, die die progressive strukturelle Herzerkrankung angeben:
Erhalten mehrerer Sätze von 6-Kanal-EKG-Daten, die dem Patienten entsprechen, aus dem an denselben Patienten angeschlossenen 6-Kanal-EKG, wobei die mehreren Sätze von 6-Kanal-EKG-Daten während voneinander beabstandeter Zeitintervalle erhalten werden, um Änderungen im Laufe der Zeit anzugeben;
Durchführung von serieller Analyse der mehreren Sätze der 6-Kanal-EKG-Daten zur Validierung der angegebenen progressiven strukturellen Herzerkrankung;
Bestimmen eines oder mehrerer Trends über die mehreren Sätze der 6-Kanal-EKG-Daten durch:
Erhalten eines ersten Herzvektors für jeden Satz der 6-Kanal-EKG-Daten;
Aufzeichnen eines Winkels jedes der ersten Herzvektoren, Erhalten einer ersten Diagnose basierend auf den Winkeln der ersten Herzvektoren, die einen Trend in einer ersten Richtung aufweisen; und
Erhalten einer zweiten Diagnose basierend auf den Winkeln der ersten Herzvektoren, die einen Trend in einer zweiten Richtung aufweisen;
und
Steuern eines Betriebs der Analysevorrichtung basierend auf einem Ergebnis der Analyse.

2. System nach Anspruch 1, wobei der mindestens eine Prozessor dazu ausgelegt ist, die Anweisungen auszuführen, um basierend auf den 12-Kanal-EKG-Daten, die die Erkrankung angeben, Folgendes auszuführen:
Erhalten eines zweiten Herzvektors für jeden Satz der 6-Kanal-EKG-Daten;
Erhalten einer Winkelbeziehung zwischen dem ersten Vektor und dem zweiten Vektor für jeden Satz der 6-Kanal-EKG-Daten; und
Detektieren eines Trends in den Winkelbeziehungen.

3. System nach Anspruch 1 oder 2, wobei der mindestens eine Prozessor dazu ausgelegt ist, die Anweisungen auszuführen, um basierend auf den 12-Kanal-EKG-Daten, die die Erkrankung angeben, Folgendes auszuführen:
Erhalten eines ersten Werts, der einem ersten quantifizierbaren Merkmal entspricht, aus jedem Satz der mehreren Sätze von 6-Kanal-EKG-Daten;
Erhalten eines ersten Trendwerts basierend auf einem Trend in den ersten Werten; und
Generieren eines Werts, der einen Grad einer Erkrankung angibt, basierend auf dem ersten Trendwert.

4. System nach Anspruch 3, wobei der mindestens eine Prozessor dazu ausgelegt ist, die Anweisungen auszuführen, um basierend auf den 12-Kanal-EKG-Daten, die die Erkrankung angeben, Folgendes auszuführen:
Vergleichen des ersten Trendwerts mit einem vordefinierten Schwellenwert; und
basierend darauf, dass der erste Trendwert den vordefinierten Schwellenwert überschreitet, Ausgeben des ersten Trendwerts.

5. System nach Anspruch 4, wobei der mindestens eine Prozessor dazu ausgelegt ist, die Anweisungen auszuführen, um basierend auf den 12-Kanal-EKG-Daten, die die Erkrankung angeben, Folgendes auszuführen:
Erhalten eines zweiten Werts, der eine zweite quantifizierbare Metrik angibt, aus jedem Satz der mehreren Sätze von 6-Kanal-EKG-Daten;
Detektieren eines zweiten Trends in den erhaltenen zweiten Werten; und
Generieren eines Werts, der den Grad der Erkrankung angibt, basierend auf dem detektierten ersten Trend und dem detektierten zweiten Trend.

6. System nach Anspruch 5, wobei der erste Trendwert auf einem ersten Merkmal in den 6-Kanal-EKG-Daten basiert, und die zweiten Trendwerte auf einem zweiten Merkmal in den 6-Kanal-EKG-Daten basieren.

7. System nach einem der vorhergehenden Ansprüche, wobei der mindestens eine Prozessor dazu ausgelegt ist, die Anweisungen auszuführen, um basierend auf den 12-Kanal-EKG-Daten, die die Erkrankung angeben, eine Diagnose zu erhalten, indem die Ergebnisse der seriellen Analyse in einen Algorithmus eingegeben werden, der dazu ausgelegt ist, progressive strukturelle Herzerkrankung basierend auf Trends in 6-Kanal-EKG-Daten zu diagnostizieren.

8. System nach einem der vorhergehenden Ansprüche, wobei der mindestens eine Prozessor dazu ausgelegt ist, die Anweisungen auszuführen, um basierend auf den 12-Kanal-EKG-Daten, die die Erkrankung angeben, Folgendes auszuführen:
Erhalten von Patientensymptomdaten, die jedem Satz der mehreren Sätze der 6-Kanal-EKG-Daten entsprechen.

9. System nach Anspruch 8, wobei der mindestens eine Prozessor dazu ausgelegt ist, die Anweisungen auszuführen, um basierend auf den 12-Kanal-EKG-Daten, die die Erkrankung angeben, Folgendes auszuführen:
Erhalten eines Symptomtrendwerts basierend auf einer seriellen Analyse eines oder mehrerer Patientensymptome, die den mehreren Sätzen von 6-Kanal-EKG-Daten entsprechen, um einen Trend in den Patientensymptomen über die Zeit zu detektieren.

10. System nach Anspruch 9, wobei der mindestens eine Prozessor dazu ausgelegt ist, die Anweisungen auszuführen, um basierend auf den 12-Kanal-EKG-Daten, die die Erkrankung angeben, eine Diagnose zu erhalten, indem die Ergebnisse der seriellen Analyse der 6-Kanal-EKG-Daten und ein Ergebnis der seriellen Analyse der Patientensymptomdaten in einen Algorithmus eingegeben werden, der dazu ausgelegt ist, eine progressive strukturelle Herzerkrankung basierend auf einem Trend in 6-Kanal-EKG-Daten und einem Trend in den Patientensymptomen zu diagnostizieren.

## Revendications

1. Système de diagnostic d'une cardiopathie structurelle progressive, comprenant :
un ECG à 12 dérivations configuré pour générer des données d'ECG à 12 dérivations, l'ECG à 12 dérivations comprenant :
10 électrodes ; et
un moniteur configuré pour mesurer des signaux électriques à travers les électrodes ;
un électrocardiogramme à 6 dérivations, ECG, configuré pour générer des données d'électrocardiogramme à 6 dérivations, l'ECG à 6 dérivations comprenant :
3 électrodes ; et
un moniteur configuré pour mesurer des signaux électriques à travers les électrodes ;
et
un dispositif d'analyse comprenant :
une mémoire (230) stockant des instructions ; et
au moins un processeur (220) configuré pour exécuter les instructions afin de :
obtenir, à partir de l'ECG à 12 dérivations relié à un patient, les données ECG à 12 dérivations correspondant à un patient ;
analyser, par un algorithme IA, les données ECG à 12 dérivations pour détecter les signes de la cardiopathie structurelle progressive ;
sur la base des données ECG à 12 dérivations indiquant la cardiopathie structurelle progressive :
obtenir, à partir de l'ECG à 6 dérivations relié au même patient, de multiples ensembles de données ECG à 6 dérivations correspondant au patient, les multiples ensembles de données ECG à 6 dérivations étant obtenus pendant des intervalles de temps espacés pour montrer les changements dans le temps ;
effectuer une analyse en série sur les multiples ensembles des données ECG à 6 dérivations pour valider la cardiopathie structurelle progressive indiquée ;
déterminer une ou plusieurs tendances sur les multiples ensembles des données ECG à 6 dérivations par :
l'obtention d'un premier vecteur cardiaque pour chaque ensemble des données ECG à 6 dérivations ;
l'enregistrement d'un angle de chacun des premiers vecteurs cardiaques,
l'obtention d'un premier diagnostic basé sur les angles des premiers vecteurs cardiaques présentant une tendance dans une première direction, et
l'obtention d'un second diagnostic basé sur les angles des premiers vecteurs cardiaques présentant une tendance vers une seconde direction,
et
commander une opération du dispositif d'analyse sur la base d'un résultat de l'analyse.

2. Système selon la revendication 1, l'au moins un processeur étant configuré pour exécuter les instructions afin de, sur la base des données d'ECG à 12 dérivations indiquant la maladie :
obtenir, pour chaque ensemble des données ECG à 6 dérivations, un second vecteur cardiaque ;
obtenir, pour chaque ensemble des données d'ECG à 6 dérivations, une relation angulaire entre le premier vecteur et le second vecteur ; et
détecter une tendance dans les relations angulaires.

3. Système selon la revendication 1 ou 2, l'au moins un processeur étant configuré pour exécuter les instructions afin de, sur la base des données d'ECG à 12 dérivations indiquant la maladie :
obtenir, à partir de chaque ensemble des multiples ensembles de données ECG à 6 dérivations, une première valeur correspondant à une première caractéristique quantifiable ;
obtenir une première valeur de tendance sur la base d'une tendance dans les premières valeurs ; et
générer une valeur indiquant un degré d'une maladie sur la base de la première valeur de tendance.

4. Système selon la revendication 3, l'au moins un processeur étant configuré pour exécuter les instructions afin de, sur la base des données d'ECG à 12 dérivations indiquant la maladie :
comparer la première valeur de tendance à un seuil prédéfini ; et
sur la base de la première valeur de tendance dépassant le seuil prédéfini, émettre en sortie la première valeur de tendance.

5. Système selon la revendication 4, l'au moins un processeur étant configuré pour exécuter les instructions afin de, sur la base des données d'ECG à 12 dérivations indiquant la maladie :
obtenir, à partir de chaque ensemble des multiples ensembles de données ECG à 6 dérivations, une seconde valeur indiquant une seconde mesure quantifiable ;
détecter une seconde tendance dans les secondes valeurs obtenues, et
générer une valeur indiquant le degré de la maladie sur la base de la première tendance détectée et de la seconde tendance détectée.

6. Système selon la revendication 5, la première valeur de tendance étant basée sur une première caractéristique dans les données ECG à 6 dérivations et les secondes valeurs de tendance étant basées sur une seconde caractéristique dans les données ECG à 6 dérivations.

7. Système selon l'une quelconque des revendications précédentes, l'au moins un processeur étant configuré pour exécuter les instructions afin de, sur la base des données ECG à 12 dérivations indiquant la maladie, obtenir un diagnostic en entrant les résultats de l'analyse en série dans un algorithme configuré pour diagnostiquer une cardiopathie structurelle progressive sur la base des tendances dans les données ECG à 6 dérivations.

8. Système selon l'une quelconque des revendications précédentes, l'au moins un processeur étant configuré pour exécuter les instructions afin de, sur la base des données d'ECG à 12 dérivations indiquant la maladie :
obtenir des données de symptômes du patient correspondant à chaque ensemble des multiples ensembles des données ECG à 6 dérivations.

9. Système selon la revendication 8, l'au moins un processeur étant configuré pour exécuter les instructions afin de, sur la base des données d'ECG à 12 dérivations indiquant la maladie :
obtenir une valeur de tendance des symptômes sur la base d'une analyse en série d'un ou plusieurs symptômes du patient correspondant aux multiples ensembles de données ECG à 6 dérivations afin de détecter une tendance dans les symptômes du patient dans le temps.

10. Système selon la revendication 9, l'au moins un processeur étant configuré pour exécuter les instructions afin de, sur la base des données ECG à 12 dérivations indiquant la maladie, obtenir un diagnostic en entrant les résultats de l'analyse en série des données ECG à 6 dérivations et un résultat de l'analyse en série des données de symptôme du patient dans un algorithme configuré pour diagnostiquer une cardiopathie structurelle progressive sur la base d'une tendance dans les données ECG à 6 dérivations et d'une tendance dans les symptômes du patient.
